# EUROPÄISCHE PATENTSCHRIFT

(11) **EP 2 704 603 B1**
(45) Veröffentlichungstag und Bekanntmachung des Hinweises auf die Patenterteilung: **17.09.2014**
(21) Anmeldenummer: 13725287.0
(22) Anmeldetag: 02.04.2013
(51) Int. Cl.: A45D 44/22, A61F 13/00

(54) **Verwendung eines Dekolletépads**
Use of a cleavage pad
Utilisation d'un coussinet de décolleté

(30) Priorität: 29.06.2012 DE 102012013010
(43) Veröffentlichungstag der Anmeldung: 12.03.2014
(73) Patentinhaber: Rickauer, Evelyn, 83233 Bernau (DE)
(72) Erfinder: Rickauer, Evelyn, 83233 Bernau (DE)
(74) Vertreter: Andrae | Westendorp Patentanwälte Partnerschaft
(86) Internationale Anmeldenummer: PCT/EP2013/056955
(87) Internationale Veröffentlichungsnummer: WO 2014/000899

(56) Entgegenhaltungen:
- WO-A1-00/69379
- DE-U1- 20 122 238
- US-A- 2 001 862
- US-A1- 2004 138 699
- US-A1- 2005 186 885

## Beschreibung

Die Erfindung betrifft die Verwendung eines Dekolletépads zum Kleben auf die Haut im Dekolletébereich der Brust.

Pads oder Klebepflaster zum Kleben auf die menschliche Haut, um damit einer Faltenbildung zu begegnen, sind allgemein bekannt. Aus US 5,961,986 ist ein Klebepad bekannt, dass zur Verminderung von Falten auf die Brust der Person geklebt wird. In WO 2005/018351 A2 wird ein Antifalten-Büstenhalter für den Schlaf offenbart. US 2008/0280534A1 zeigt eine Vorrichtung zum Stecken zwischen die Brüste zur Vermeidung von Falten in der Brustregion und aus CA 2575534 ist ein klebefähiges Pad zum Formen der Brust bekannt. Die Dekolletépads werden dabei auf die Bereiche geklebt, an denen die Faltenbildung auftreten kann, oder bereits aufgetreten ist.

Die Dekolletépads basieren auf der Erkenntnis, dass Falten im Dekolletébereich durch eine Stauchung der Haut über einen längeren Zeitraum, wie z.B. während des Schlafens auf einer Seite, verursacht oder zumindest begünstigt werden. Die Stauchung der Haut wird hier, wie auch im Folgenden, als eine Stauchung der Haut in der Ebene der Hautoberfläche verstanden. Dieses Problem findet gerade bei dem weiblichen Dekolleté Beachtung.

Im Bereich zwischen den Brüsten und dem unmittelbar darüber liegenden Bereich liegt im Allgemeinen eine besonders starke Faltenbildung vor. Diese wird dadurch verursacht oder verstärkt, da während des Schlafens beim Seitenliegen die untere Brust durch Auflage auf dem unteren Arm oder auf der Matratze sich nicht nach unten bewegt oder ggf. durch den unteren Arm sogar etwas angehoben wird, wo hingegen die obere Schulter und die obere Brust sich aufgrund der Schwerkraft nach unten absenken. Im mittleren Bereich des Dekolletés, also in einem Bereich, der in vertikaler Sicht etwa gleich weit von den Brustwarzen und den Schlüsselbeinen entfernt ist, liegt eine weitgehend gleichmäßige Verteilung der Falten vor.

Die bekannten Dekolletépads weisen eine Form auf, die daraufhin optimiert wurde, dass alle Stellen, an denen die Faltenbildung auftreten kann, von dem Pad abgedeckt sind. Da die Steifigkeit der bekannten Pads derart hoch ist, dass beim seitlichen Liegen der Person ein Stauchen des Pads selbst praktisch nicht, oder nur in einem vernachlässigbarem Maße, auftritt und die darunter liegende Haut mit dem Pad verklebt ist, tritt aufgrund des Pads keine Stauchung der verklebten Haut auf und der Faltenbildung wird entgegen gewirkt. Diese Steifigkeit der bekannten Pads bewirkt allerdings, dass das Tragegefühl für die Person teilweise unangenehm sein kann. Ein Grund für das unangenehme Tragegefühl kann darin liegen, dass aus dem Stand der Technik bekannte Pads eine Verlängerung haben, der im Zwischenbrustbereich direkt zwischen den Brüsten liegt, da dort die Faltenbildung besonders ausgebildet sein kann. Es kann beim Liegen auf der Seite die obere Brust auf der nach oben gerichteten Kante dieser Verlängerung des Pads aufliegen und aufgrund der erhöhten Empfindlichkeit der Brust ein unangenehmes Gefühl verursachen.

Aufgabe der vorliegenden Erfindung ist es, bekannte Dekolletépads in ihrer Antifaltenwirkung bei der Verwendung im Dekolletébereich der Brust zu verbessern und dabei bei der Trägerin ein möglichst angenehmes Tragegefühl zu bewirken. Diese Aufgabe wird durch die Merkmale des unabhängigen Anspruchs gelöst. Vorteilhafte Weiterbildungen ergeben sich aus den Unteransprüchen.

Ein Dekolletépad kann auf die Haut geklebt werden und weist eine Stützschicht und eine Klebeschicht auf, wobei das Dekolletépad spiegelsymmetrisch zu einer Symmetrieachse ist. Dabei wird die Außenkontur des Dekolletépads durch eine im Wesentlichen konkave Kontur und eine im Wesentlichen konvexe Kontur mit Rundungen in den Übergangen beider Konturen gebildet, wobei ein Schnittpunkt A der konvexen Kontur mit der Symmetrieachse und ein Schnittpunkt B der konkaven Kontur mit der Symmetrieachse besteht, und sich über eine lotrechte Projektion des Punkts der Rundungen des größten Abstands vom Schnittpunkt A auf die Symmetrieachse ein Punkt C ergibt. Das Verhältnis der Stecke AB / AC ist kleiner als 0,8 und beträgt bevorzugt 0,6. Über dieses Verhältnis ergibt sich, dass das Dekolletépad eine Art Halsausschnitt aufweist. Anders formuliert kann man bevorzugt obiges Verhältnis auch so beschreiben, dass das Pad zu beiden Seiten in der Nähe der Schlüsselbeine endet. So wird erreicht, dass sich eine Gesamtform ergibt, die einen Großteil des oberen Dekolletés abdeckt. In dem abgeklebten Bereich wird eine deutlich erhöhte Steifigkeit des Dekolletés gegen Stauchen erreicht. Da der abgeklebte Bereich eine relativ große Fläche aufweist wird auch bewirkt, dass das gesamte Dekolleté und nicht nur die abgeklebten Bereiche wirksam gegen ein Zusammenstauchen und eine entsprechende Faltenbildung geschützt sind. Somit kann auf eine Beklebung des Zwischenbrustbereichs verzichtet werden. Da gerade hier bei bekannten Ausführungsformen der Grund für ein unangenehmes Tragegefühl begründet war, wird der Tragekomfort erhöht.

Insbesondere liegt die konkave Kontur mit einer Toleranz von +/- 2 cm auf einem ersten Kreisbogen, wobei insbesondere der Radius des ersten Kreisbogens 9,5 cm beträgt. Nach einer bevorzugten Ausführungsform beträgt der Radius des ersten Kreisbogens 9 cm. Durch die konkave Kreisbogenkontur kann das Pad in einem gleich bleibenden Abstand zum Hals so angebracht werden. Dabei enden die oberen Abschnitte des Pads kurz unterhalb der Schlüsselbeinknochen.

Weiterführend liegt die konvexe Kontur mit einer Toleranz von +/- 3 cm auf einem zweiten Kreisbogen und liegt vorzugsweise mit der Toleranz von +/- 2,5 cm, insbesondere mit der Toleranz von +/- 2 cm, auf dem zweiten Kreisbogen, wobei insbesondere der Radius des zweiten Kreisbogens 11 cm beträgt. Nach einer bevorzugten Ausführungsform beträgt der Radius des zweiten Kreisbogens 10 cm. Dadurch wird ausgedrückt, dass das Pad im Wesentlichen sichelförmig ausgestaltet und im Wesentlichen im Dekolleté oberhalb der Brüste und weniger im Zwischenbrustbereich angeordnet ist.

Ein Punkt A der konvexen Kontur liegt auf der Symmetrieachse des Dekolletépads und die konvexe Kontur liegt mit einer Toleranz von +/- 3 cm auf einem zweiten Kreisbogen. Dabei besteht die konvexe Kontur in einem vom Mittelpunkt des zweiten Kreisbogens ausgehenden Bogenwinkelbereich der konvexen Kontur von +/- 35°, insbesondere +/-30°, um den Schnittpunkt A aus Radien, die größer als der Radius des zweiten Kreisbogens sind. Insbesondere sind die Radien um höchstens 2 cm kleiner, als der Radius des zweiten Kreisbogens. Hier liegt ein systematischer Unterschied zum Stand der Technik vor. Vormals waren Ausführungsformen bekannt, bei denen das Pad relativ spitz in die Zwischenbrustfalte ausläuft. Vorliegend ist der Radius aber eher abgeflacht, wodurch sich die bereits erläuterten Vorteile ergeben.

Weiter kann die Außenkontur des Dekolletépads eine im Wesentlichen konvexe Kontur umfassen, wobei ein Schnittpunkt der konvexen Kontur mit der Symmetrieachse des Dekolletépads besteht und der Radius eines zweiten Kreisbogens mindestens 8 cm, insbesondere mindestens 9 cm beträgt und die konvexe Kontur in einem Kreiswinkelbereich von +/- 35° um den Schnittpunkt innerhalb eines Toleranzbereich von +/- 1 cm um den zweiten Kreisbogen liegt. Entsprechend wird über diese Beschreibung ausgedrückt, dass das Dekolletépad oberhalb des Zwischenbrustbereichs abgeflacht ist und keinen Fortsatz aufweist, der in den Zwischenbrustbereich geführt ist.

Insbesondere weist das Dekolletépads eine Größe auf, dass in dem Dekolletépad ein rechteckiger und senkrecht zur Symmetrieachse ausgerichteter Bereich mit den Maßen mindestens 14,5 cm * 3,5 cm einschreibbar ist. Nach einer bevorzugten Ausführungsform weist das Dekolletépad eine Größe auf, dass in dem Dekolletépad ein rechteckiger und senkrecht zur Symmetrieachse ausgerichteter Bereich mit den Maßen mindestens 15 cm * 4 cm einschreibbar ist. Bei den Pads des Stands der Technik wurde gezielt und praktisch ausschließlich der Bereich abgeklebt, bei dem verstärkt die Falten auftreten, also insbesondere keilförmig in den Zwischenbrustbereich. Es wurde jedoch erkannt, dass ein horizontal aufgeklebtes Band der genannten Maße der Haut im gesamten Dekolleté derart Halt geben kann, dass dadurch auch benachbarte Hautbereiche, die nicht abgeklebt sind vor einer Faltenbildung geschützt sind. Das Dekolletépad ist größer als die genannten Maße, um den Effekt weiter zu unterstützen.

Vorteilhafter Weise weist das Decolletépad einen außen umlaufenden Rand der Breite von 2 mm +/-1 mm auf, bei dem die Dicke des Dekolletépads gegenüber dem Rest deutlich reduziert ist. Hierdurch wird ein äußerer Bereich geringerer Festigkeit geschaffen, um Druckstellen der Kante an der Brust zu verringern oder zu vermeiden.

Auch kann das Decolletépad einen umlaufenden Rand der Breite von 2 mm +/-1,5 mm aufweisen, bei dem die Dicke des Dekolletépads gegenüber dem Rest um mindestens 60% reduziert ist, wodurch ebenfalls ein Randbereich geringerer Festigkeit geschaffen wird, um Druckstellen zu reduzieren. Der Rand kann so dünn und/oder flexibel sein, dass er sich gegenüber dem Pad abwinkeln kann und dass die Brust an dem abgewinkelten Bereich anliegt, wodurch über einer Vergrößerung der Anlagefläche weiter ein möglicherweise unangenehmes Gefühl reduziert werden kann.

Auch kann an dem Pad ein außen umlaufender Rand vorgesehen sein, der frei von einer Klebebeschichtung ist, wodurch die Entfernung der Trennschicht von dem Pad erleichtert wird.

Bevorzugt beträgt die maximale Breite des Decolletépads in der Ausrichtung senkrecht zur Symmetrieachse mindestens 18 cm, wodurch ein Großteil des oberen Dekolletés abgedeckt wird. Durch die erfindungsgemäße Dimensionierung des Dekolletépads wird in überraschend günstiger Weise eine Stabilisierung der Haut in den bei der Faltenbildung relevanten Bereichen des Dekolletés ermöglicht, wobei gleichzeitig ein sehr hoher Tragekomfort sichergestellt wird.

Nach einer Ausführungsform weist ein Dekolletépad zum Kleben auf die Haut im Dekolletébereich der Brust gemäß der Patentansprüche eine Schicht aus einem Klebesilikon zur Verklebung mit dem Körper und eine bei der Verwendung des Dekolletépad vom Körper weg zeigende äußere Schicht aus einer ersten Polyurethanfolie auf. Bevorzugt sind zwischen der äußeren Schicht und der Schicht aus Klebesilikon eine zweite Polyurethanfolie und eine Silikonschicht angeordnet und die Silikonschicht ist zwischen der ersten und der zweiten Polyurethanfolie angeordnet. Dabei sind die beiden Polyurethanfolien an ihren Außenenden umlaufend verklebt oder verschweißt.

Nach einer weiteren Ausführungsform findet nur eine Kunststofffolie oder gar keine Kunststofffolie Verwendung, um den Aufbau weiter zu vereinfachen. Dabei weist vorzugsweise die Stützschicht aus Silikon eine ausreichende Stabilität auf, um - wie oben beschrieben - als Stützschicht im Sinne der Erfindung dienen zu können. Bevorzugt beträgt die Dicke 1,75 mm und bei Varianten liegt sie zwischen 1,3 und 3 mm. Die Klebeschicht kann nach einer Ausführungsform direkt auf die Stützschicht aufgebracht sein. Nach einer bevorzugten Ausführungsform besteht somit das Dekolletépad aus einer Stützschicht und einer Klebeschicht. Bevorzugt ist die Stützschicht (und vorzugsweise auch die Klebeschicht) homogen aufgebaut, d.h. insbesondere nicht aus mehreren Schichten zusammengesetzt.

Nach einer Ausführungsform kann am umlaufenden Rand die Dicke der Silikonschicht sukzessiv abnehmend reduziert sein, um in diesem Bereich eine erhöhte Elastizität zu erhalten, um so Druckstellen an der Brust zu verhindern. Nach einer Ausführungsform kann die minimale Dicke des Randes zwischen 10-80% der Gesamtdicke liegen. Zusätzlich kann nach einer Ausführungsform der umlaufende Rand der Stützschicht nicht mit der Klebeschicht versehen sein, um die Entfernung des Pads von der Trennschicht oder dem Körper zu erleichtern. In einer alternativen Ausführungsform kann auch der Rand mit der Klebeschicht versehen sein.

Die Erfindung betrifft die Verwendung des Pads wie in der vorliegenden Beschreibung und den Ansprüchen beschrieben zum Aufkleben auf die Haut im Dekolletébereich der Brust. Ein weiterer Aspekt betrifft das Aufkleben auf die Haut und das Tragen des Dekolletépads auf der Haut über Nacht, insbesondere während des Schlafens. Bevorzugt wird das Dekolletépad verwendet, um einer Faltenbildung im Bereich des Dekolletés vorzubeugen und/oder gegen Dekolletéfalten zu wirken, insbesondere bei Personen, die auf der Seite schlafen.

Durch die vorteilhafte Stabilisierung der Haut mit Hilfe des aufgeklebten Dekolletépads legt sich diese in den für die Verhinderung der Faltenbildung maßgeblichen Bereichen nicht in scharfe Falten, sondern maximal in größere, weiche Wellen. Die Verformung der Haut wird somit so minimiert, dass keine Druckfalten entstehen. Das Dekolletépad stabilisiert die dünne, feine Haut in den relevanten Bereichen des Dekolletés quasi zu einer dickeren, robusteren Haut. Dabei bleibt das Dekolletépad aufgrund seiner Dimensionierung und Beschaffenheit so anschmiegsam, dass ein hoher Tragekomfort gegeben ist. Aufgrund der Ausgestaltung des Dekolletépads ist dieser Tragekomfort auch bei unterschiedlichen bevorzugten Liege- bzw. Schlafpositionen gewährleistet.

Bevorzugt ist auf dem Dekolletépad, insbesondere auf dessen Klebeschicht, kein nichtklebendes Kissen angeordnet, wie man es z.B. bei Wundpflastern findet. Somit ist vorzugsweise eine Seite des Dekolletépads vollständig, d.h. mit ihrer ganzen Fläche, oder im Wesentlichen vollständig selbstklebend und kann so zum Tragen auf die Haut aufgeklebt werden. Nach einer Ausführungsform kann ein Randbereich gegebenenfalls frei von der Klebebeschichtung sein, wie vorstehend beschrieben.

Nachfolgend wird die Erfindung anhand von bevorzugten Ausführungsformen beschrieben. Die unter Bezugnahme auf die Figuren beschriebenen einzelnen Merkmale sind als eigenständige und kombinierbare Ausführungsformen zu verstehen, sofern es sich nicht um klare, sich gegenseitig ausschließende Alternativen handelt. Es zeigen:
- Fig. 1: ein Dekolletépad gemäß dem Stand der Technik,
- Fig. 2: eine Ansicht eines weiblichen Oberkörpers mit einem hier beschriebenen Dekolletépad,
- Fig. 3 bis Fig. 6: Ansichten des Dekolletépads mit unterschiedlichen Bemaßungen und
- Fig. 7: zeigt einen seitlichen Schnitt durch das Dekolletépad,
- Fig. 8: zeigt einen seitlichen Schnitt durch eine Variante des Dekolletépads.

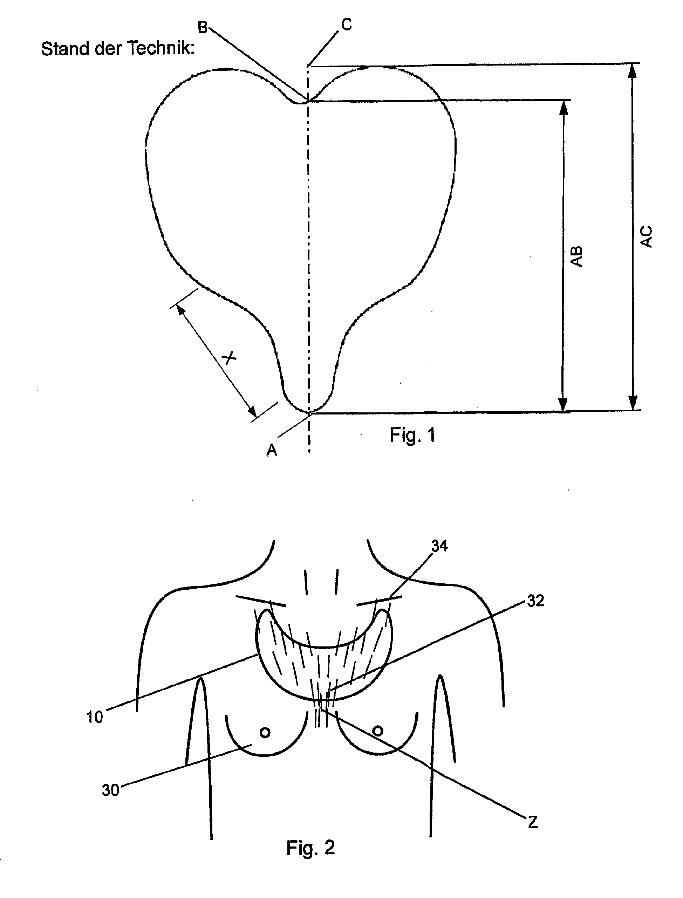

Figur 1 zeigt ein Dekolletépad des Standes der Technik. Diese Ausführungsform hat eine in etwa herzförmige Grundform. Die untere Spitze des Herzes ist so ausgestaltet, dass sie in den Zwischenraum der beiden Brüste aufgenommen wird. An den von der Spitze ausgehenden Schenkeln der herzförmigen Kontur sind leicht konkav geformte Bereiche X angeordnet. Beim Liegen auf der Seite liegt nämlich die jeweils obere Brust auf der Außenkontur des Pads auf und dies kann am empfindlichen Brustgewebe ein unangenehmes Gefühl, Druckstellen und Schmerzen verursachen. Durch diese konkave Kontur X wird beim Stand der Technik versucht, dieses Problem zu reduzieren.

Fig. 2 zeigt einen weiblichen Oberkörper mit Brüsten 30 und einem im Dekolleté angebrachten Dekolletépad 10. Ferner sind Falten 32 gezeigt, die bei Frauen häufig auftreten, wenn sie Seitenschläfer sind. Dabei sind die Falten im Zwischenbrustbereich ausgeprägter als im oberen Dekolletébereich.

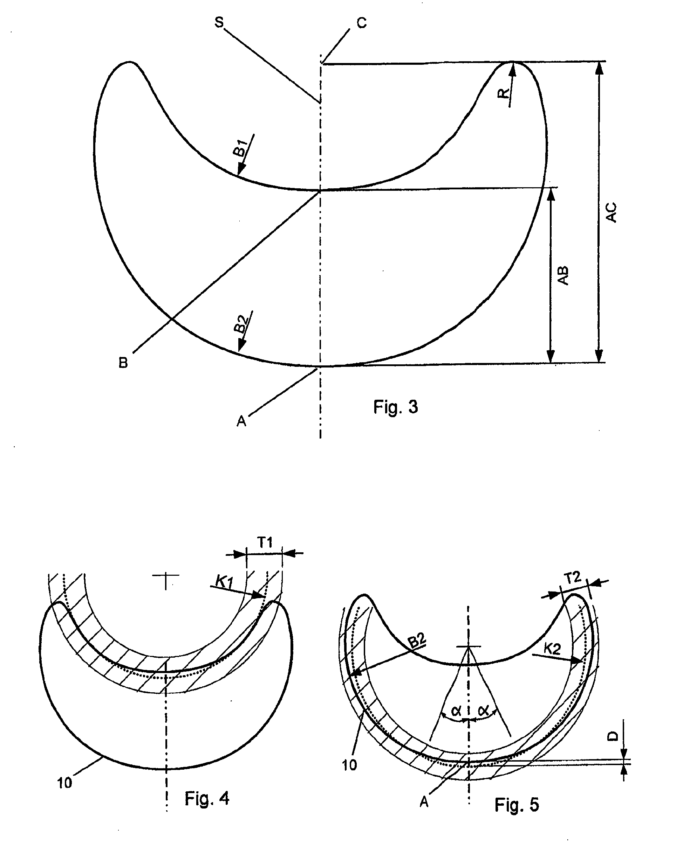

In Fig. 2 ist gezeigt, dass auf das Dekolleté ein Dekolletépad 10 aufgeklebt ist, und dieses ist in Fig. 3 vergrößert gezeigt. In horizontaler Richtung, wobei die Ausrichtung in der Verwendung bei einer stehenden Person gemeint ist, beträgt die Breite des Dekolletépad 24 cm. Das Dekolletépad 10 weist eine Sichelform oder Halbmondform auf, die im Wesentlichen aus den beiden Bögen B1 und B2 besteht, die an den Übergängen zueinander mit einem Radius R versehen sind. Zum Zweck der Erläuterung werden an dem Dekolletépad 10 die Symmetrieachse S und Bezugspunkte A bis C definiert. Die Symmetrieachse S ist die vertikale Achse, die das Dekolletépad in zwei spiegelsymmetrisch identische Teile teilt. Der Schnittpunkt der Symmetrieachse S mit dem konvexen Bogen B2 wird als A bezeichnet. Der Schnittpunkt des konkaven Bogens B1 mit der Symmetrieachse S wird als B bezeichnet. Ferner wird ein Punkt C als der Lotpunkt von dem obersten Punkt des Dekolletépads auf die Symmetrieachse definiert. Alternativ kann dieser Punkt C als die Projektion des Punktes des Dekolletépads auf die Symmetrieachse bezeichnet werden, der den maximalen Abstand von dem Punkt A hat.

Die Höhe des Dekolletépads, also die Entfernung der Punkte A bis C, beträgt 15,5 cm. Die Sichel des Pads wird dabei über zwei Bögen B1 und B2, die im Wesentlichen Kreisbögen sind, gebildet, die im Übergang zueinander eine Rundung R aufweisen. Der Radius der Rundung beträgt bevorzugt zwischen 10 und 20 mm. Der Bogen B1 beschreibt den kleineren innenliegenden und konkaven Bogen und weist in etwa die Form eines Kreisbogens mit dem Radius von 9,5 cm auf. Hierbei ist die exakte Radiusform nicht wesentlich, sondern die Kontur des Bogens B1 liegt bei einer bevorzugten Ausführungsform mit einer Abweichung von +/- 15 mm, vorzugsweise +/- 5mm, um diesem Bogen. Bei der Verwendung des Dekolletépads liegt der Bogen B1 äquidistant zu dem Hals der Trägerin. Die Enden der sichelartigen Form des Dekolletépads, also der Bereich der Radien R liegen dabei unterhalb der Schlüsselbeine der Trägerin und in einem Abstand von ca. 3 cm zu den Schlüsselbeinen. So wird bewirkt, dass unterhalb der Schlüsselbeine keine Faltenbildung auftreten kann, oder dort die Faltenbildung zumindest reduziert wird. Die Breite des Toleranzfelds des Bogens B1 ist in Fig. 4 mit T1 gekennzeichnet und bezeichnet die Abweichung, die der Bogen B1 bei erfindungsgemäßen Ausführungsformen von der idealen Kreisbogenform K1 haben kann.

Der Bogen B2 beschreibt den größeren außen liegenden und konvexen Bogen und weist in etwa die Form eines Kreisbogens mit dem Radius K2 (siehe Fig. 5) von 11,3 cm mit dem Toleranzbereich T2 auf. Dabei weicht der Bogen B2 bei einer bevorzugten Ausführungsform nicht mehr als +/- 15 mm (bevorzugt +/- 10mm) von der Kreisbogenform des Radius K2 ab.

Aus Fig. 2 ergibt sich, dass das Dekolletépad nicht den Bereich Z zwischen den Brüsten liegt. Bei der Betrachtung der Geometrie wird ersichtlich, dass die der Abstand AB wie eine Halsaussparung größer ist, als bei bekannten Ausführungsformen. Verallgemeinert ergibt sich, dass das Verhältnis der Stecken AB / AC kleiner als 0,8 ist. Dieses Verhältnis bewirkt, dass im oberen Brustbereich eine relativ große Fläche abgedeckt wird. Aufgrund der Steifigkeit des Pads wird bewirkt, dass beim Seitenliegen die Gesamtheit der Haut im oberen Brustbereich deutlich weniger abfallen kann als bei bekannten Ausführungsformen. Durch diese Flächenverteilung und die dadurch bewirkte Erhöhung der Steifigkeit der Oberbrust wird bewirkt, dass die Haut im Zwischenbrustbereich Z nicht zusammengestaucht werden kann und so wird in diesem Bereich Z einer Faltenbildung entgegengewirkt, ohne dass in diesem Bereich ein Abschnitt des Pads aufgeklebt ist.

Aus Fig. 5 ergibt sich, dass der Bogen B2 im Umfeld des Punkts A eine größeren Radius aufweist, als der Radius des Kreisbogens K2. So ergibt sich bei dem Punkt A eine Distanz D zwischen dem Kreisbogen K2 und dem Bogen B2. Somit ist das Dekolletépad oberhalb des Zwischenbrustbereichs Z in seiner Außenkontur stärker abgeflacht, als es der idealen Kreisbogenform K2 entsprechen würde. Entsprechend ist der Zwischenbrustbereich Z frei von dem Dekolletépad und die Gesamtsteifigkeit des Pads bewirkt, dass die Haut sich im Zwischenbrustbereich nicht zusammenstauchen kann und hierdurch wird auch ohne ein Aufkleben des Pads im Zwischenbrustbereich Z verhindert, dass hier die Haut beim Liegen auf der Seite Falten ausbilden kann.

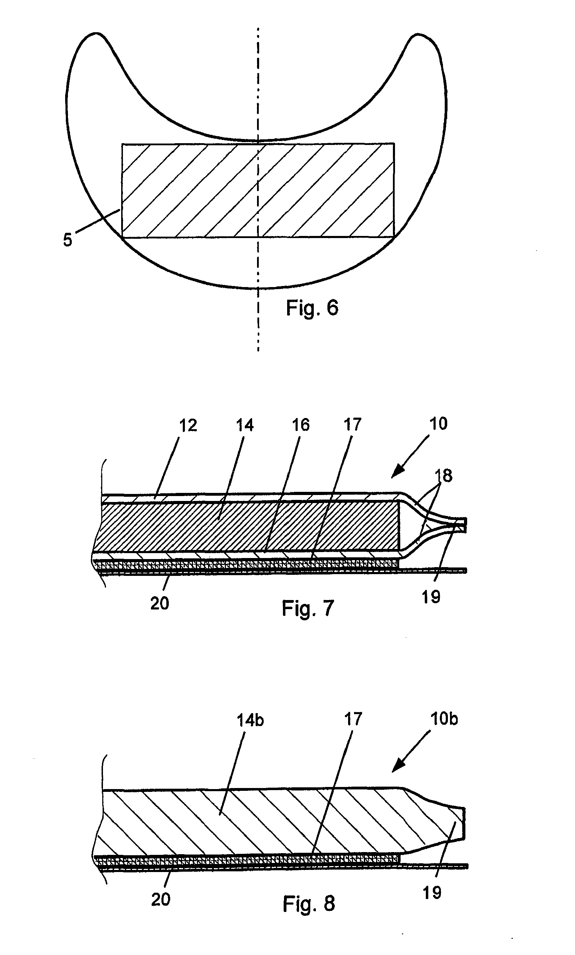

Fig. 6 dient ergänzend zur Erläuterung des schematischen Aufbaus des Pads. Die Fläche des Pads ist derart groß, dass in ihm ein Rechteck der Größe von 19,5 cm * 5 cm enthalten ist. Dieses Rechteck liegt als Band oberhalb der Brüste und sorgt dafür, dass die Haut im Oberbrustbereich nicht zusammengestaucht werden kann. Die übrigen Bereiche des Pads unterstützen diese Wirkung.

Aus Fig. 7 ergibt sich der strukturelle Aufbau des Dekolletépads 10. Zwischen einer äußeren ersten Kunststofffolie 12 und einer unteren zweiten Kunststofffolie 16 liegt eine Silikonschicht 14, so dass sich hierdurch ein Sandwich-Aufbau ergibt. Beide Kunststofffolien 12 und 16 sind bevorzugt gleich dick und haben eine Dicke von 10µm bis 200 µm und bevorzugt eine Dicke von 100 µm +/- 30 µm und sind aus einem Thermoplastkunststoff, wie bspw. Polyurethan gefertigt. Die dazwischen liegende Silikonschicht 14 hat eine maximale Schichtdicke von 5mm und bevorzugt eine Schichtdicke von 1,5 mm +/- 1 mm. Die Festigkeit der Silikonschicht beträgt 5 Shore 00 - 65 Shore 00. Dieser Sandwich-Aufbau bewirkt zum einen, dass die beiden Kunststofffolien 12 und 16 von einander beabstandet sind, wodurch die Festigkeit des Pads gegen ein seitliches Stauchen erhöht ist. Zum anderen bleibt aber eine Biegbarkeit des Dekolletépads erhalten, so dass das mit der Brust verklebte Dekolletépad sich der Körperkontur anpassen kann.

Zum Verkleben des Dekolletépads mit der Haut ist auf der Außenseite der zweiten Kunststofffolie ist eine Klebeschicht 17 aus einem Klebesilikon der Dicke von 0,2 mm aufgebracht, wobei die Dicke alternativ bis zu 5 mm betragen kann.

Die Silikonschicht 14 ist eine aus einer Folie gestanzte Schicht mit Schnittkanten und somit Stirnflächen, die senkrecht zur Folie liegen. Die Kunststofffolien 12 und 16 sind um 2 mm größer als die Silikonschicht 14, so dass sich ein umlaufender Rand 18 bildet. Am außen liegenden Ende 19 des Rands 18 sind die Kunststofffolien 12 und 16 miteinander verschweißt. Die Klebeschicht 17 hat in etwa die Größe der Silikonschicht 14. Da im Randbereich 18 zwischen den Folien 12 und 16 kein Silikon enthalten ist, ist er deutlich weicher als der Rest des Dekolletépads. Beim Seitenliegen der Trägerin können Brüste gegen den Rand 18 drücken. Dieser verformt sich und es ergibt sich eine vergrößerte Kontaktfläche mit der Brust, so dass ein unangenehmes Gefühl vermieden wird.

Auf der Unterseite der Klebeschicht 17 ist eine Trennschicht 20 aus einer dünnen Kunststofffolie oder Wachspapier angeordnet, die sich leicht von der Klebeschicht 17 trennen lässt. Da die Trennschicht am Rand 18 nicht mit dem Dekolletépad verbunden ist, lässt sie sich einfach von der Anwenderin entfernen und anschließend wird das Dekolletépad mit der Klebeschicht 17 auf das Dekolleté aufgeklebt.

In Fig. 8 ist eine Variante des Dekolletépads mit einem veränderten Aufbau gezeigt. Hier finden keine Kunststoffolien 12, 16 Verwendung. Stattdessen weist die Silikonschicht 14b eine ausreichende Stabilität auf, um - wie oben beschrieben - als Stützschicht dienen zu können. Bevorzugt beträgt die Dicke 1,75 mm und bei Varianten liegt sie zwischen 1,5 und 3 mm. Am umlaufenden Rand 18 der Breite von 2mm ist die Dicke der Silikonschicht sukzessiv abnehmend reduziert, um in diesem Bereich eine erhöhte Elastizität zu erhalten, um so Druckstellen an der Brust zu verhindern. Die minimale Dicke des Randes liegt zwischen 40-80% der Gesamtdicke Zusätzlich kann optional der umlaufende Rand 18 nicht mit der Klebeschicht 17 versehen sein, um die Entfernung des Pads von der Trennschicht 20 zu erleichtern. In einer alternativen Ausführungsform kann auch der Rand 18 mit der Klebeschicht versehen sein. In diesem Fall ragt die Trennschicht 20 in vorteilhafter Weise über den Rand 18 hinaus (in Fig. 8 nicht dargestellt), um so die Trennschicht zum Entfernen von dem Pad leicht greifen zu können.

Bei der Erstellung der Figuren wurde auf die Proportionalität der Figuren geachtet, so dass weitere, in der Beschreibung nicht explizit aufgeführte bevorzugte Größen- und/oder Winkelverhältnisse den Figuren entnehmbar sind.

## Patentansprüche

1. Verwendung eines Dekolletépads (10) zum Kleben auf die Haut im Dekolletebereich der Brust, mit einer Stützschicht (12, 14, 14b, 16) und einer Klebeschicht (17), wobei das Dekolletépad (10) spiegelsymmetrisch entlang einer Symmetrieachse (S) ist, wobei die Außenkontur des Dekolletépads durch eine im Wesentlichen konkave Kontur (B1) und eine im Wesentlichen konvexe Kontur (B2) mit Rundungen (R) in den Übergangen beider Konturen gebildet ist, **dadurch gekennzeichnet, dass** ein Schnittpunkt A der konvexen Kontur (B1) mit der Symmetrieachse (S) und ein Schnittpunkt B der konkaven Kontur (B2) mit der Symmetrieachse (S) besteht, und sich über eine lotrechte Projektion des Punkts der Rundungen (R) des größten Abstands vom Schnittpunkt A auf die Symmetrieachse (S) ein Punkt C ergibt, und das Verhältnis der Stecke AB / AC kleiner als 0,8 ist.

2. Verwendung eines Dekolletépads gemäß Anspruch 1, **dadurch gekennzeichnet, dass** die konkave Kontur (B1) mit einer Toleranz (T1) von +/- 2 cm auf einem ersten Kreisbogen (K1) liegt, wobei insbesondere der Radius des ersten Kreisbogens (K1) 9 cm +/- 2,5 cm, insbesondere 9,5 cm +/- 2 cm beträgt.

3. Verwendung eines Dekolletépads gemäß Anspruch 1 oder 2, **dadurch gekennzeichnet, dass** die konvexe Kontur (B2) mit einer Toleranz (T2) von +/- 3 cm auf einem zweiten Kreisbogen (K2) und vorzugsweise mit der Toleranz von +/-2,5 cm, insbesondere +/- 2 cm auf dem Kreisbogen (K2) liegt, wobei insbesondere der Radius des zweiten Kreisbogens 11 cm +/- 1 cm beträgt.

4. Verwendung eines Dekolletépads gemäß Anspruch 1 oder 2, **dadurch gekennzeichnet, dass** die konvexe Kontur (B2) mit einer Toleranz (T2) von +/- 3 cm auf einem zweiten Kreisbogen (K2) und vorzugsweise mit der Toleranz von +/-2,5 cm, insbesondere +/- 2 cm auf dem Kreisbogen (K2) liegt, wobei insbesondere der Radius des zweiten Kreisbogens 10 cm +/- 2 cm beträgt.

5. Verwendung eines Dekolletépads (10) nach einem der vorstehenden Ansprüche, mit einer Stützschicht (12, 14, 14b, 16) und einer Klebeschicht (17), wobei das Dekolletépad (10) spiegelsymmetrisch entlang einer Symmetrieachse (S) ist, **dadurch gekennzeichnet, dass**
die Außenkontur des Dekolletépads eine im Wesentlichen konvexe Kontur (B2) umfasst, wobei ein Schnittpunkt (A) der konvexen Kontur mit der Symmetrieachse (S) des Dekolletépads (10) besteht und der Radius eines zweiten Kreisbogens (T2) mindestens 8 cm, insbesondere mindestens 9 cm beträgt und die konvexe Kontur (B2) in einem Winkelbereich (α) des Kreisbogens von +/- 35° um den Schnittpunkt (A) innerhalb eines Toleranzbereich (T2) von +/- 1 cm um den zweiten Kreisbogen (T2) liegt.

6. Verwendung eines Dekolletépads gemäß einem der vorangegangenen Ansprüche, **dadurch gekennzeichnet, dass** das Dekolletépads eine Größe aufweist, dass in dem Dekolletépad ein rechteckiger und senkrecht zur Symmetrieachse (S) ausgerichteter Bereich (5) mit den Maßen 14,5 cm * 3,5 cm, insbesondere 15 cm * 4 cm, enthalten ist.

7. Verwendung eines Dekolletépads gemäß einem der vorangegangenen Ansprüche, **dadurch gekennzeichnet, dass** das Decolletépad einen umlaufenden Rand der Breite von 2 mm +/-1 mm aufweist, bei dem die Dicke des Dekolletépads gegenüber dem Rest um mindestens 20% reduziert ist.

8. Verwendung eines Dekolletépads gemäß einem der vorangegangenen Ansprüche, **dadurch gekennzeichnet, dass** das Decolletépad einen umlaufenden Rand (18) der Breite von 2 mm +/-1,5 mm aufweist, bei dem die Dicke des Dekolletépads gegenüber dem Rest um mindestens 60% reduziert ist.

9. Verwendung eines Dekolletépads gemäß Anspruch 7 oder 8, **dadurch gekennzeichnet, dass** das an dem umlaufenden Rand (18) keine Klebebeschichtung (17) vorgesehen ist.

10. Verwendung eines Dekolletépads gemäß einem der vorangegangenen Ansprüche, **dadurch gekennzeichnet, dass** die maximale Breite des Decolletépads in der Ausrichtung senkrecht zur Symmetrieachse (S) mindestens 18 cm beträgt.

11. Verwendung eines Dekolletépads gemäß einem der vorangegangenen Ansprüche, **dadurch gekennzeichnet, dass** das Dekolletépad aus einer Stützschicht und einer Klebeschicht besteht, wobei eine oder vorzugsweise beide dieser Schichten homogen aufgebaut sind.

12. Verwendung eines Dekolletépads gemäß einem der vorangegangenen Ansprüche, **dadurch gekennzeichnet, dass** das Dekolletépad (10) eine Schicht aus einem Klebesilikon (17) zur Verklebung mit dem Körper und eine bei der Verwendung des Dekolletépad eine vom Körper weg zeigende äußere Schicht (12) aus einer ersten Kunststofffolie, insbesondere einer Polyurethanfolie, aufweist.

13. Verwendung eines Dekolletépads gemäß Anspruch 12, **dadurch gekennzeichnet, dass** zwischen der äußeren Schicht (12) und der Schicht aus Klebesilikon (17) eine zweite Kunststofffolie (16), insbesondere eine Polyurethanfolie und eine Silikonschicht (14) angeordnet sind und die Silikonschicht (14) zwischen der ersten (12) und der zweiten (16) Kunststofffolie angeordnet ist.

14. Verwendung eines Dekolletépads gemäß Anspruch 12 oder 13, **dadurch gekennzeichnet, dass** die beiden Kunststofffolien (12, 16) an ihren Außenenden (19) umlaufend verklebt oder verschweißt sind.

15. Verwendung eines Dekolletépads gemäß einem der vorstehenden Ansprüche, um bei Personen, die auf der Seite schlafen, einer Faltenbildung im Bereich des Dekolletés vorzubeugen und/oder gegen Dekolletéfalten zu wirken.

## Claims

1. A use of a décolleté pad (10) for adhering on the skin in the décolleté region of the chest with a backing layer (12, 14, 14b, 16) and an adhesive layer (17), wherein the décolleté pad (10) shows mirror-symmetry along a symmetry axis (S), wherein the overall contour of the décolleté pad is formed by an essentially concave contour (B1) and an essentially convex contour (B2) with curvatures (R) in the areas of contact of both contours, **characterized in that** there is a point of intersection A of the convex contour (B1) with the symmetry axis (S) and a point of intersection B of the concave contour (B2) with the symmetry axis (S) and that a point C results from a perpendicular projection of the point of the curvatures (R) with the greatest distance from the point of intersection A onto the symmetry axis (S) and that the ratio of the length of AB / AC is smaller than 0.8.

2. The use of a décolleté pad according to claim 1, **characterized in that** the concave contour (B1) describes a first circular arc (K1) with a tolerance (T1) of +/- 2 cm, wherein in particular the radius of the first circular arc (K1) is 9 cm +/- 2.5 cm, in particular 9.5 cm +/- 2 cm.

3. The use of a décolleté pad according to claim 1 or 2, **characterized in that** the convex contour (B2) describes a second circular arc (K2) with a tolerance (T2) of +/- 3 cm, and preferably the circular arc (K2) with the tolerance of +/- 2.5 cm, in particular +/- 2 cm, wherein in particular the radius of the second circular arc is 11 cm +/- 1 cm.

4. The use of a décolleté pad according to claim 1 or 2, **characterized in that** the convex contour (B2) describes a second circular arc (K2) with a tolerance (T2) of +/- 3 cm, and preferably the circular arc (K2) with the tolerance of +/- 2.5 cm, in particular +/- 2 cm, wherein in particular the radius of the second circular arc is 10 cm +/- 2 cm.

5. The use of a décolleté pad (10) according to one of the preceding claims with a backing layer (12, 14, 14b, 16) and an adhesive layer (17), wherein the décolleté pad (10) shows mirror-symmetry along a symmetry axis (S), **characterized in that** the overall contour of the décolleté pad comprises an essentially convex contour (B2), wherein there is a point of intersection (A) of the convex contour with the symmetry axis (S) of the décolleté pad (10) and the radius of a second circular arc (T2) is at least 8 cm, in particular at least 9 cm and the convex contour (B2) within an angle range (α) of the circular arc of +/- 35° around the point of intersection (A) is within a tolerance range (T2) of +/- 1 cm with respect to the second circular arc (T2).

6. The use of a décolleté pad according to one of the preceding claims, **characterized in that** the décolleté pad has such a size that the décolleté pad comprises a rectangular area (5) being arranged perpendicularly with respect to the symmetry axis (S) with dimensions of 14.5 cm * 3.5 cm, in particular 15 cm * 4 cm.

7. The use of a décolleté pad according to one of the preceding claims, **characterized in that** the décolleté pad has a circumferential edge with a width of 2 mm +/- 1 mm, wherein there the thickness of the décolleté pad is reduced in an extent of at least 20 % with respect to the remainder of the décolleté pad.

8. The use of a décolleté pad according to one of the preceding claims, **characterized in that** the décolleté pad has a circumferential edge (18) with a width of 2 mm +/- 1.5 mm, wherein there the thickness of the décolleté pad is reduced in an extent of at least 60 % with respect to the remainder of the décolleté pad.

9. The use of a décolleté pad according to claim 7 or 8, **characterized in that** at the circumferential edge (18) no adhesive coating (17) is provided.

10. The use of a décolleté pad according to one of the preceding claims, **characterized in that** the maximum width of the décolleté pad arranged in the direction perpendicularly to the symmetry axis (S) is at least 18 cm.

11. The use of a décolleté pad according to one of the preceding claims, **characterized in that** the décolleté pad consists of a backing layer and an adhesive layer, wherein one of or preferably both of these layers are homogenously designed.

12. The use of a décolleté pad according to one of the preceding claims, **characterized in that** the décolleté pad (10) comprises a layer of an adhesive silicone (17) for adhering to the body and an outer layer (12) of a first plastic foil, in particular a polyurethane foil, being arranged at the far side with respect to the body during the use of the décolleté pad.

13. The use of a décolleté pad according to claim 12, **characterized in that** between the outer layer (12) and the layer of adhesive silicone (17) a second plastic foil (16), in particular a polyurethane foil and a silicone layer (14) are arranged and that the silicone layer (14) is arranged between the first (12) and the second (16) plastic foils.

14. The use of a décolleté pad according to claim 12 or 13, **characterized in that** both plastic foils (12, 16) are circumferentially adhered or fused at their external ends (19).

15. The use of a décolleté pad according to one of the preceding claims for doing something against décolleté wrinkles and/or preventing the formation of wrinkles in the region of the décolleté of persons who sleep on their sides.

## Revendications

1. Utilisation d'un coussinet de décolleté (10), avec une couche de soutien (12, 14, 14b, 16) et une couche adhésive (17), sachant que le coussinet de décolleté (10) est en symétrie spéculaire le long d'un axe de symétrie (S), le contour extérieur du coussinet de décolleté étant formé par un contour sensiblement concave (B1) et un contour sensiblement convexe (B2) avec des arrondis (R) dans les jonctions des deux contours, **caractérisée en ce qu**'il existe un point d'intersection (A) entre le contour concave (B1) et l'axe de symétrie (S) et un point d'intersection (B) entre le contour convexe (B2) et l'axe de symétrie (S) et qu'un point (C) est formé sur l'axe de symétrie (S), au-dessus d'une projection perpendiculaire du point des arrondis (R) du plus grand intervalle du point d'intersection (A), et que le rapport de la distance AB / AC est inférieur à 0,8.

2. Utilisation d'un coussinet de décolleté selon la revendication 1,
**caractérisée en ce que** le contour concave (B1) est situé sur un premier arc de cercle (K1) avec une tolérance (T1) de +/- 2 cm, sachant particulièrement que le rayon du premier arc de cercle (K1) est de 9 cm +/- 2,5 cm, en particulier de 9,5 cm +/- 2 cm.

3. Utilisation d'un coussinet de décolleté selon revendication 1 ou 2,
**caractérisée en ce que** le contour convexe (B2) est situé, avec une tolérance (T2) de +/- 3 cm, sur un deuxième arc de cercle (K2), et est de préférence situé sur le deuxième arc de cercle (K2) avec une tolérance de +/- 2,5 cm, en particulier de +/- 2 cm, sachant particulièrement que le rayon du deuxième arc de cercle (K2) est de 11 cm +/- 1 cm.

4. Utilisation d'un coussinet de décolleté selon revendication 1 ou 2,
**caractérisée en ce que** le contour convexe (B2) est situé, avec une tolérance (T2) de + / - 3 cm, sur un deuxième arc de cercle (K2) et est de préférence situé sur le deuxième arc de cercle (K2) avec une tolérance de +/- 2,5 cm, en particulier de +/- 2 cm, sachant particulièrement que le rayon du deuxième arc de cercle est de 10 cm +/- 2 cm.

5. Utilisation d'un coussinet de décolleté (10) selon l'une des revendications précédentes, avec une couche de soutien (12, 14, 14b, 16) et une couche adhésive (17), sachant que le coussinet de décolleté (10) est en symétrie spéculaire le long d'un axe de symétrie (S),
**caractérisée en ce que** le contour extérieur du coussinet de décolleté est doté d'un contour sensiblement convexe (B2), sachant qu'il existe un point d'intersection (A) du contour convexe avec l'axe de symétrie (S) du coussinet de décolleté (10) et que le rayon d'un deuxième arc de cercle (K2) est d'au moins 8 cm, en particulier d'au moins 9 cm, et que le contour convexe (B2) est situé dans une zone angulaire (α) de l'arc de cercle de +/- 35 ° par rapport au point d'intersection (A), dans une plage de tolérance (T2) de + / - 1 cm par rapport au deuxième arc de cercle (K2).

6. Utilisation d'un coussinet de décolleté selon l'une des revendications précédentes,
**caractérisée en ce que** le coussinet de décolleté est dimensionné de sorte que ledit coussinet de décolleté enceigne une zone (5) de forme rectangulaire et orientée perpendiculairement à l'axe de symétrie (S), qui présente des dimensions de 14,5 cm * 3,5 cm, en particulier de 15 cm * 4 cm.

7. Utilisation d'un coussinet de décolleté selon l'une des revendications précédentes
**caractérisée en ce que** ledit coussinet de décolleté est doté d'un bord périphérique d'une largeur de 2 mm +/- 1 mm, dans lequel l'épaisseur du coussinet de décolleté est réduite d'au moins 20 % par rapport au reste.

8. Utilisation d'un coussinet de décolleté selon l'une des revendications précédentes.
**caractérisée en ce que** ledit coussinet de décolleté est doté d'un bord périphérique (18) d'une largeur de 2 mm +/- 1,5 mm, dans lequel l'épaisseur du coussinet de décolleté est réduite d'au moins 60 % par rapport au reste.

9. Utilisation d'un coussinet de décolleté selon revendication 7 ou 8,
**caractérisée en ce que**, sur le bord périphérique (18), n'est prévue aucune couche adhésive (17).

10. Utilisation d'un coussinet de décolleté selon l'une des revendications précédentes,
**caractérisée en ce que** la largeur maximale du coussinet de décolleté est d'au moins 18 cm en orientation perpendiculaire à l'axe de symétrie (S).

11. Utilisation d'un coussinet de décolleté selon l'une des revendications précédentes,
**caractérisée en ce que** le coussinet de décolleté consiste en une couche de soutien et une couche adhésive, sachant que l'une ou, de préférence, les deux couches sont de constitution homogène.

12. Utilisation d'un coussinet de décolleté selon l'une des revendications précédentes,
**caractérisée en ce que** le coussinet de décolleté (10) est doté d'une couche de silicone adhésive (17) pour l'adhésion au corps et d'une couche extérieure (12) consistant en une première feuille de matière synthétique, de préférence une feuille de polyuréthane qui, lors de l'utilisation du coussinet de décolleté, est orientée à l'opposé du corps.

13. Utilisation d'un coussinet de décolleté selon la revendication 12,
**caractérisée en ce que**, entre la couche extérieure (12) et la couche de silicone adhésive (17), sont disposées une deuxième feuille de matière synthétique (16), en particulier une feuille de polyuréthane, et une couche de silicone (14), et que la couche de silicone (14) est disposée entre la première feuille (12) et la deuxième feuille (16) en matière synthétique.

14. Utilisation d'un coussinet de décolleté selon revendication 12 ou 13,
**caractérisée en ce que** les deux feuilles de matière synthétique (12, 16) sont collées ou soudées en continu à leurs extrémités extérieures (19).

15. Utilisation d'un coussinet de décolleté selon l'une des revendications précédentes, destiné à prévenir, chez les personnes dormant sur le côté, la formation de plis dans la région du décolleté et / ou pour combattre les plis du décolleté.
